# EUROPÄISCHE PATENTSCHRIFT

(11) **EP 2 494 979 B1**
(45) Veröffentlichungstag und Bekanntmachung des Hinweises auf die Patenterteilung: **27.03.2019**
(21) Anmeldenummer: 12156640.0
(22) Anmeldetag: 23.02.2012
(51) Int. Cl.: A61K 36/16, A61P 9/10

(54) **Verwendung von Extrakten aus Blättern von Ginkgo biloba zur Prävention und Therapie von Komplikationen nach operativer Arteriosklerose-Behandlung sowie von Folgeerscheinungen der Arteriosklerose**
Use of ginkgo biloba leaf extract to prevent and treat complications following arteriosclerosis surgery and reappearance of arteriosclerosis
Utilisation d'extraits de feuilles de ginkgo biloba pour la prévention et la thérapie de complications après un traitement post-opératoire de l'artériosclérose ainsi qu'effets secondaires de l'artériosclérose

(30) Priorität: 04.03.2011 DE 102011005097
(43) Veröffentlichungstag der Anmeldung: 05.09.2012
(73) Patentinhaber: Dr. Willmar Schwabe GmbH & Co. KG, 76227 Karlsruhe (DE)
(72) Erfinder: Siegel, Günter, 12203 Berlin (DE); Koch, Egon, 76229 Karlsruhe (DE)
(74) Vertreter: Adam, Holger

(56) Entgegenhaltungen:
- WO-A1-2005/046519
- US-B1- 6 187 314
- G. SIEGEL ET AL: "Hs-CRP may be associated with white blood cell count in metabolic syndrome patients treated with Ginkgo biloba", ATHEROSCLEROSIS, Bd. 218, Nr. 1, 1. September 2011 (2011-09-01), Seiten 250-252, XP055023333, ISSN: 0021-9150, DOI: 10.1016/j.atherosclerosis.2011.05.007
- SOO LIM ET AL: "EGb761, a Ginkgo Biloba Extract, Is Effective Against Atherosclerosis In Vitro, and in a Rat Model of Type 2 Diabetes", PLOS ONE, Bd. 6, Nr. 6, 2. Juni 2011 (2011-06-02), Seite E20301, XP055023336, DOI: 10.1371/journal.pone.0020301
- GÜNTER SIEGEL ET AL: "Ginkgo biloba (EGb 761) in arteriosclerosis prophylaxis", WIENER MEDIZINISCHE WOCHENSCHRIFT, Bd. 157, Nr. 13-14, 1. Juli 2007 (2007-07-01), Seiten 288-294, XP019522516, SPRINGER-VERLAG, AT ISSN: 1563-258X, DOI: 10.1007/S10354-007-0426-6
- RODRIGUEZ ET AL: "Reduction of atherosclerotic nanoplaque formation and size by Ginkgo biloba (EGb 761) in cardiovascular high-risk patients", ATHEROSCLEROSIS, Bd. 192, Nr. 2, 1. Juni 2007 (2007-06-01), Seiten 438-444, XP022289902, ELSEVIER IRELAND LTD, IE ISSN: 0021-9150, DOI: 10.1016/J.ATHEROSCLEROSIS.2007.02.021
- GÃ NTER SIEGEL ET AL: "The importance of scavenging reactive oxygen species in anti-aging medicine", ENGINEERING IN LIFE SCIENCES, Bd. 9, Nr. 5, 1. Oktober 2009 (2009-10-01) , Seiten 363-375, XP055023338, ISSN: 1618-0240, DOI: 10.1002/elsc.200800112
- FENYE LIU ET AL: "Inhibitory effect ofGinkgo Biloba extract on hyperhomocysteinemia-induced intimal thickening in rabbit abdominal aorta after balloon injury", PHYTOTHERAPY RESEARCH, Bd. 22, Nr. 4, 1. Januar 2008 (2008-01-01) , Seiten 506-510, XP055023340, ISSN: 0951-418X, DOI: 10.1002/ptr.2353
- SHING-JONG LIN ET AL: "Effects of Ginkgo biloba extract on the proliferation of vascular smooth muscle cells in vitro and on intimal thickening and interleukin-1? expression after balloon injury in cholesterol-fed rabbits in vivo", JOURNAL OF CELLULAR BIOCHEMISTRY, Bd. 85, Nr. 3, 1. Januar 2002 (2002-01-01) , Seiten 572-582, XP055023343, ISSN: 0730-2312, DOI: 10.1002/jcb.10151
- PIETRI SYLVIA ET AL: "Ginkgo biloba extract (EGb 761) pretreatment limits free radical-induced oxidative stress in patients undergoing coronary bypass surgery", CARDIOVASCULAR DRUGS AND THERAPY, Bd. 11, Nr. 2, 1997, Seiten 121-131, XP002672598, ISSN: 0920-3206
- SHEN JIANGANG ET AL: "Ginkgo biloba extract (EGb761) inhibits mitochondria-dependent caspase pathway and prevents apoptosis in hypoxia-reoxygenated cardiomyocytes.", CHINESE MEDICINE, Bd. 6, 8, 23. Februar 2011 (2011-02-23), Seiten 1-9, XP002672599, ISSN: 1749-8546
- LIU H J ET AL: "Inhibitions of vascular endothelial growth factor expression and foam cell formation by EGb 761, a special extract of Ginkgo biloba, in oxidatively modified low-density lipoprotein-induced human THP-1 monocytes cells", PHYTOMEDICINE, Bd. 16, Nr. 2-3, 1. März 2009 (2009-03-01) , Seiten 138-145, XP025968638, GUSTAV FISCHER VERLAG, STUTTGART, DE ISSN: 0944-7113, DOI: 10.1016/J.PHYMED.2008.11.003 [gefunden am 2009-01-08]

## Beschreibung

Die vorliegende Erfindung betrifft Extrakte aus Blättern von Ginkgo biloba zur oralen Verwendung zur Prävention und Therapie von Komplikationen nach operativer Arteriosklerose-Behandlung, wobei es sich um die Beschleunigung der verzögerten Reendothelisierung nach der Implantation eines beschichteten Stents handelt, oder wobei es sich bei den Komplikationen nach operativer Arteriosklerose-Behandlung um Stent-induzierte Thrombosen oder um die Restenose von verengten Arterien nach der Implantation von unbeschichteten und beschichteten Stents handelt. Bei dem Extrakt handelt es sich besonders bevorzugt um den Ginkgo-Extrakt EGb 761®.

Erkrankungen des Herz-Kreislaufsystems gehören in den westlichen Industrienationen mit Abstand zu den wichtigsten Todesursachen. Einen ganz wesentlichen Anteil daran hat die koronare Herzkrankheit (KHK) an deren Folgemanifestationen (z.B. Herzinfarkte und plötzlicher Herztod) in der Bundesrepublik jährlich mehr als 100.000 Menschen versterben.

Wichtigste auslösende Ursache für eine KHK ist eine Arteriosklerose der Herzkranzgefäße. Bei der Arteriosklerose (auch Atherosklerose, umgangssprachlich auch Arterienverkalkung) handelt es sich um eine chronisch-degenerative Erkrankung der Arterienwände. Der Krankheitsprozess beginnt am Endothel und erfasst sukzessiv die gesamte Intima und die Media. In der Arterienwand kommt es zur Ablagerung von Lipiden, der Proliferation von glatten Muskelzellen und zur Bildung von Bindegewebe. Die Gefäße verlieren an Elastizität und das Gefäßlumen wird zunehmend enger. In der Folge kommt es zu Durchblutungsstörungen und der Gefahr eines vollständigen Gefäßverschlusses. Die klinisch wichtigsten Manifestationen der Arteriosklerose sind koronare Herzkrankheit (KHK), Myokardinfarkt, Schlaganfall, periphere arterielle Durchblutungsstörungen (pAVK, Claudicatio intermittens) und Aneurysmen.

Als Risikofaktoren für die Ausbildung einer Arteriosklerose gelten vor allem eine Hyperlipidämie, Hypertonie, Nikotinabusus und Hyperhomocysteinämie. Am Beginn der Erkrankung steht eine Schädigung des Endothels, die mit einer Erhöhung der Gefäßpermeabilität einhergeht, so dass vermehrt Low Density Lipoproteine (LDL) infiltrieren können. Im subendothelialen Raum unterliegen die nativen LDL einer oxidativen Schädigung (ox-LDL) durch reaktive Sauerstoffspezies (ROS), die vor allem aus dem Stoffwechsel von Leukozyten, Endothelzellen und Myozyten stammen. Ox-LDL regt Endothel- und Muskelzellen zur Bildung von chemotaktisch wirksamen Slgnalstoffen (MCP-1, MCSF-1) an, die die Einwanderung von zirkulierenden Monozyten in die Arterienwand und deren Transformation zu Makrophagen fördern. Ausdifferenzierte Makrophagen produzieren vermehrt Superoxidanionen und verstärken dadurch die Oxidation von LDL, die mit einem Verlust der strukturellen Integrität von Apo B einhergeht. In dieser Form verändertes LDL wird nach Bindung an Scavenger-Rezeptoren von Makrophagen internalisiert. Da der Scavenger-Rezeptor im Gegensatz zum LDL-Rezeptor durch hohes intrazelluläres ox-LDL nicht down-reguliert wird, häuft sich Cholesterin in den Makrophagen an, die dann als Schaumzellen bezeichnet werden. In der Folge bilden sich sogenannte Fettstreifen (fatty streaks) und schließlich atherogene Plaques, die eine zusätzliche Endothelschädigung mit weiterer Einwanderung von Makrophagen und LDL in den subendothelialen Raum auslösen. Ox-LDL erhöhen außerdem durch einen direkt toxischen Effekt auf das Endothel die Gefäßpermeabilität. Monozyten und ausdifferenzierte Makrophagen setzen zusätzlich Wachstumsfaktoren (Interleukine, Platelet derived growth factor [PDGF]) und Eicosanoide (Thromboxan B2, Leukotrien B4 und C4) frei. Diese stimulieren die Einwanderung, Transformation und Aktivität von Monozyten, induzieren die Proliferation und Migration von glatten Muskelzellen und verstärken die Aggregation von Thrombozyten. Mit fortschreitendem Prozess bilden sich ausgedehnte Ansammlungen von Schaumzellen, Fettstreifen und Bindegewebswucherungen, wodurch sich das Lumen der betroffenen Gefäßabschnitte zunehmend einengt (Hahn et al., Ernährungs-Umschau 49: 172, 2002).

Als Folge einer Arteriosklerose der Koronargefäße kommt es durch die Beeinträchtigung der Organdurchblutung zu einer verminderten Sauerstoffversorgung der Herzmuskulatur. Das Leitsymptom der KHK ist die Angina pectoris (Brustenge). Mit zunehmendem Fortschreiten der Erkrankung erhöht sich die Wahrscheinlichkeit für das Auftreten von klinischen Folgeerkrankungen, wie Herzrhythmusstörungen und Herzinsuffizienz, sowie akuten, lebensbedrohlichen Komplikationen wie Herzinfarkt und plötzlicher Herztod.

Neben Umstellungen im Lebensstil (z.B. Nikotinabstinenz, Veränderung der Ernährungsgewohnheiten) und der medikamentösen Therapie (z.B. Thrombozytenaggregationshemmer, Nitrate, β-Blocker, Ca-Antagonisten, Lipidsenker) zählen bei fortgeschrittenen Krankheitszuständen die perkutane transluminale Koronarangioplastie (PTCA, Ballondilatation) oder die aortokoronare Bypass-Operation zum therapeutischen Standardrepertoire (Köster et al., Med. Monatsschr. Pharm. 23, 149, 2000) bei der KHK.

Seit ihrer Einführung im Jahre 1977 (Grüntzig et al., N. Engl. J. Med. 12, 61,1979) hat die Zahl der Ballonkatheterdilatationen dramatisch zugenommen. Während in Deutschland im Jahre 1998 etwa 158.000 Eingriffe durchgeführt wurden, werden weltweit jährlich vermutlich mehr als 1.000.000 Koronarangioplastien vorgenommen. Durch Verbesserung der Kathetertechnologie und der Interventionstechnik hat sich die Indikationsstellung für die PTCA deutlich erweitert und sie kann häufig auch bei Patienten mit Mehrgefäßerkrankungen und Komorbidität angewendet werden (Köster et al., Med. Monatsschr. Pharm. 23, 149, 2000, Schiele et al. Dtsch. Med. Wochenschr. 126, 440, 2001). Im Vergleich zur Bypass-Operation ist die Ballondilatation in den ersten Jahren nach dem Eingriff kostengünstiger und hat hinsichtlich der Überlebensrate und der Vermeidung von Myokardinfarkten eine ähnliche Prognose. Die Vorteile der PTCA werden aber durch eine vermehrte Inzidenz von Angina pectoris-Beschwerden und vor allem durch eine hohe Rate von Restenosen begrenzt (Henderson et al, Lancet 352, 1419, 1998). Während nach einfacher Ballondilatation unkomplizierter Gefäßverengungen Restenoseraten von 30-60 % beobachtet werden, wird die Zahl der erneuten Stenosen durch gleichzeitige Implantation eines Stents zwar verringert, sie liegt aber immer noch bei 10 bis 40 % (Bult, Trends Pharmacol. Sci. 21, 274, 2000; Köster et al., Med. Monatsschr. Pharm. 23, 149, 2000; van der Hoeven et al., Int. J. Cardiol. 99, 9, 2005).

Durch die Implantation eines Stents wird die nach einer Ballondilatation auftretende elastische Wiederverengung des geweiteten Gefäßes verhindert, die Ausbildung einer sogenannten Neointima wird aber nicht verhindert. Ausgelöst wird diese durch eine entzündliche Reaktion als Folge der Dehnung und mechanischen Schädigung der Gefäßwand. Initial werden Thrombozyten aktiviert, die sich an die Gefäßwand und das Metallgerüst des Stents anhaften, gefolgt von der Adhäsion von Leukozyten. Durch die Freisetzung von Zytokinen und Wachstumsfaktoren wird die Migration und Proliferation von glatten Muskelzellen stimuliert und es kommt zur Ausformung einer Neointima. Das Neointimavolumen ist in der Regel 3 bis 6 Monate nach der Stentimplantation maximal ausgeprägt. Anschließend werden die glatten Muskelzellen zunehmend durch die Ablagerung einer extrazellulären Matrix ersetzt.

Aufgrund der großen klinischen Relevanz von Restenosen und den erheblichen Kosten für eine erneute Revaskularisierung wurde intensiv an der Entwicklung von technischen und medikamentösen Konzepten zur Verhinderung dieses Prozesses gearbeitet (Topol und Serruys, Circulation 98, 1802, 1998). Zu den bisher untersuchten pharmakologischen Substanzen zur Restenoseprophylaxe zählen Thrombozytenaggregationshemmer, Cholesterinsenker, Gerinnungshemmer, ACE-Inhibitoren, Entzündungshemmer, Immunsuppressiva und antiproliferative Verbindungen. Bisher konnte in klinischen Studien für keines der geprüften Medikamente aber eine überzeugende therapeutische Wirksamkeit nachgewiesen werden bzw. die Anwendungen waren mit schwerwiegenden systemischen Nebenwirkungen verbunden (de Feyter et al., Curr. Interv. Cardiol. Rep. 2, 326, 2000; van der Hoeven et al., 2005).

Der Wunsch, die Vorteile eines Stents zu nutzen und gleichzeitig die Ausbildung einer Neointima durch Einsatz von proliferationshemmenden Medikamenten unter Vermeidung derer systemischen Nebenwirkungen zu verhindern, führte zur Entwicklung von beschichteten Stents (drug eluting stents), die kleine Mengen von Arzneimitteln über einen längeren Zeitraum freisetzen. Im Wesentlichen haben sich seit deren Einführung im Jahre 2002 für die Beschichtung zwei Wirkstoffe durchgesetzt: das Immunsuppressivum Sirolimus und das Zytostatikum Paclitaxel.

Die aktuelle Datenlage zum Vergleich der unterschiedlichen Stentarten und auch zur Bypass-Operation ist nicht eindeutig und die Diskussion hierüber ist nicht abgeschlossen. Metaanalysen zeigen bezüglich der Sterblichkeitsrate von Patienten keinen signifikanten Unterschied zwischen medikamentenfreisetzenden Stents und unbeschichteten Stents, wenngleich eine erhöhte Stentthrombose-Rate im Langzeitverlauf bei Paclitaxel-Stents beobachtet wurde (Stettler et al., Lancet 370, 937, 2007). Obwohl die Restenoserate bei beschichteten Stents niedriger ist, hat die Begeisterung in den letzten Jahren doch deutlich nachgelassen, da insgesamt ein Trend zu einer höheren Mortalitätsrate besteht. Außerdem wird eine fehlende Reendothelisierung beobachtet und es kommt beim Absetzen von Thrombozytenaggregationshemmern auch noch viele Monate nach der Implantation zur Bildung von Thrombosen.

Es wird momentan empfohlen, medikamentenfreisetzende Stents bevorzugt bei erhöhtem Risiko einer Restenose (wie bei Diabetikern), jedoch zurückhaltend bei erhöhtem Risiko einer Stentthrombose einzusetzen. Medikamentenfreisetzende Stents sollen nicht eingesetzt werden, wenn die Möglichkeit einer verlängerten Gabe eines Thrombozytenaggregationshemmers, z. B. wegen einer anstehenden chirurgischen Behandlung, nicht gegeben ist oder wenn damit zu rechnen ist, dass der Patient die Medikation nicht einhält (Zylka-Menhorn, Dtsch Arztebl 104, 2007; A-1272, 2007).

Aufgrund der vorausgehenden Ausführungen besteht weiterhin ein ausgepragtes Interesse und Bedürfnis an oral zu verabreichenden und nebenwirkungsarmen Wirkstoffen zur Verhinderung einer Restenose wie auch grundsätzlich der Behandlung der KHK. Ansatzpunkte für neue Präventions- und Behandlungsmethoden liefern aktuelle wissenschaftliche Untersuchungen. Während in den 1970er Jahren die zentrale Bedeutung von Lipiden bei der Betrachtung der Pathogenese der Arteriosklerose vorherrschte, rückte in den 1980er Jahren die Rolle von Wachstumsfaktoren und die Proliferation von glatten Muskelzellen verstärkt in den Focus. In den folgenden Jahren setzte sich vermehrt die Überzeugung durch, dass es sich bei Arteriosklerose um eine chronische Entzündungs- und Immunreaktion handelt (Libby, Nature 420, 868, 2002). Neueste wissenschaftliche Untersuchungen deuten darauf hin, dass eosinophile Granulozyten einen ganz wesentlichen Anteil am Krankheitsgeschehen haben. Mehrere prospektive Studien zeigten einen Zusammenhang zwischen der Zahl der Eosinophilen im Blut und dem Risiko für ein kardiovaskuläres Ereignis. Außerdem fanden sich erhöhte Konzentrationen von Eotaxin, einem Eosinophilen-spezifischen Chemokin, in atherosklerotischen Plaques und im Serum von Patienten mit KHK. In Übereinstimmung damit gibt es Hinweise, dass ein Polymorphismus des Eotaxingens und eine Sequenzvariante für die Eosinophilenzahl mit einem erhöhten Risiko für einen Myokardinfarkt korrelieren (Niccoli et al., Atherosclerosis 211, 606, 2010).

Offenbar sind Eosinophile auch maßgeblich an der Restenose und an Thrombosen bei Patienten mit beschichteten Stents beteiligt. So fanden sich in der Leukozytenpopulation, die beschichtete Stents infiltrierte, im Vergleich zu unbeschichtenen Stents ein höherer Anteil von Eosinophilen. In einer prospektiven Studie wurde nachgewiesen, dass die Plasma-Konzentration von Eosinophilen Cationic Protein (ECP) im Serum von Patienten mit beschichteten Stents einen hohen Vorhersagewert für das Auftreten eines schweren kardialen Ereignisses hat (Niccoli et al., Eur. Heart J. 30, 1340, 2009). Diese Ergebnisse implizieren, dass Allergievermittelte Immunreaktionen offenbar sowohl an der Pathogenese der Arteriosklerose als auch an der Restenose nach der Implantation von insbesondere beschichteten Stents beteiligt sind und eröffnen neue Möglichkeiten für die Prophylaxe und Therapie der KHK.

In Ergänzung zu den vorstehend genannten Veröffentlichungen wird noch auf folgende Veröffentlichungen verwiesen: G. SIEGEL ET AL, "Hs-CRP may be associated with white blood cell count in metabolic syndrome patients treated with Ginkgo biloba", ATHEROSCLEROSIS, Bd. 218, Nr. 1, 1. September 2011, Seiten 250-252; SOO LIM ET AL, "EGb761, a Ginkgo Biloba Extract, Is Effective Against Atherosclerosis In Vitro, and in a Rat Model of Type 2 Diabetes", PLOS ONE, Bd. 6, Nr. 6, 2. Juni 2011, Seite E20301; US 6,187,314 B1; GÜNTER SIEGEL ET AL, "Ginkgo biloba (EGb 761) in arteriosclerosis prophylaxis", WIENER MEDIZINISCHE WOCHENSCHRIFT, SPRINGER-VERLAG, AT, Bd. 157, Nr. 13-14, 1. Juli 2007, Seiten 288-294; RODRIGUEZ ET AL, "Reduction of atherosclerotic nanoplaque formation and size by Ginkgo biloba (EGb 761) in cardiovascular high-risk patients", ATHEROSCLEROSIS, ELSEVIER IRELAND LTD, IE, Bd. 192, Nr. 2, 1. Juni 2007, Seiten 438-444; GÜNTER SIEGEL ET AL, "The importance of scavenging reactive oxygen species in anti-aging medicine", ENGINEERING IN LIFE SCIENCES, Bd. 9, Nr. 5, 1. Oktober 2009, Seiten 363-375; FENYE LIU ET AL, "Inhibitory effect of Ginkgo Biloba extract on hyperhomocysteinemia-induced intimal thickening in rabbit abdominal aorta after balloon injury", PHYTOTHERAPY RESEARCH, Bd. 22, Nr. 4, 1. Januar 2008, Seiten 506-510; SHING-JONG LIN ET AL, "Effects of Ginkgo biloba extract on the proliferation of vascular smooth muscle cells in vitro and on intimal thickening and interleukin-1β expression after balloon injury in cholesterol-fed rabbits in vivo", JOURNAL OF CELLULAR BIOCHEMISTRY, Bd. 85, Nr. 3, 1. Januar 2002, Seiten 572-582; PIETRI SYLVIA ET AL, "Ginkgo biloba extract (EGb 761) pretreatment limits free radical-induced oxidative stress in patients undergoing coronary bypass surgery", CARDIOVASCULAR DRUGS AND THERAPY, Bd. 11, Nr. 2, 1997, Seiten 121-131; SHEN JIANGANG ET AL, "Ginkgo biloba extract (EGb761) inhibits mitochondriadependent caspase pathway and prevents apoptosis in hypoxia-reoxygenated cardiomyocytes", CHINESE MEDICINE 2011 LNKD-PUBMED:21345217, Bd. 6, 23. Februar 2011, Seite 8; LIU H J ET AL, "Inhibitions of vascular endothelial growth factor expression and foam cell formation by EGb 761, a special extract of Ginkgo biloba, in oxidatively modified low-density lipoprotein-induced human THP-1 monocytes cells", PHYTOMEDICINE, GUSTAV FISCHER VERLAG, STUTTGART, DE, Bd. 16, Nr. 2-3, 1. März 2009, Seiten 138-145 und WO 2005/046519 A1.

### Beispiel:

In einer Pilotstudie an Patienten mit metabolischem Syndrom und einem erhöhten Arteriosklerose-Risiko wurde jetzt überraschend beobachtet, dass die Verabreichung des Ginkgo biloba-Spezialextraktes EGb 761® die Zahl sowohl der Monozyten als auch der Eosinophilen im peripheren Blut signifikant verringerte. Der Extrakt ist in Deutschland zugelassen zur Behandlung von dementiellem Syndrom bei primär degenerativer Demenz, vaskulärer Demenz und Mischformen, peripherer arterieller Verschlusskrankheit sowie Vertigo und Tinnitus vaskulärer und involutiver Genese. Durch die jetzt gemachte überraschende Beobachtung ergeben sich neue Möglichkeiten für die Verwendung von Extrakten aus Blättern von Ginkgo biloba zur Prävention und Therapie von Komplikationen nach operativer Arteriosklerose-Behandlung, wobei es sich um die Beschleunigung der verzögerten Reendothelisierung nach der Implantation eines beschichteten Stents handelt, oder wobei es sich bei den Komplikationen nach operativer Arteriosklerose-Behandlung um Stent-induzierte Thrombosen oder um die Restenose von verengten Arterien nach der Implantation von unbeschichteten und beschichteten Stents handelt.

Ergebnisse dieser Pilotstudie an Patienten mit metabolischem Syndrom wurden teilweise bereits publiziert (Siegel et al., Eng. Life Sci. 9, 363, 2009). Es handelte sich um eine präventive, randomisierte, 12-wöchige Studie mit einer 4-wöchigen run-in-Phase, gefolgt von einer 8-wöchigen Behandlungsperiode. Elf Patienten (2 Männer, 9 Frauen) im Alter von 26-48 Jahren mit metabolischem Syndrom wurden in die Studie aufgenommen, vorausgesetzt, sie erfüllten zusätzliche Einschlußkriterien, wie Rauchen (alle Patienten) und eine Blut-Lipoprotein(a)-Konzentration >30 mg/dL (9 Patienten). Nach einer ersten Blutabnahme vor Therapiebeginn erfolgte die Behandlung mit 2 x 120 mg/Tag Ginkgo biloba Spezialextrakt EGb 761® über zwei Monate. Es wurden keine Statine, keine Calcium-Antagonisten und keine Nitratverbindungen verabreicht. Es traten keine Nebenwirkungen auf und alle Patienten fühlten sich während und nach der Ginkgo-Behandlung wohl. Nach 2-monatiger Medikation erfolgte die Abnahme einer zweiten Blutprobe.

In einer aktuellen Re-Evaluierung zusätzlich gemessener klinischer Laborparameter ergab sich ein überraschender Befund (Tabelle 1). Neben einer Senkung der Gesamtzahl weißer Blutkörperchen ergab sich eine rund 65%-ige Reduktion der Monozyten sowie eine rund 43%-ige Verminderung der Eosinophilen. Auf der Grundlage dieser Befunde kann festgestellt werden, daß die antiarteriosklerotische Wirkung einer Monozytenabnahme und die anti-allergische und entzündungshemmende Wirkung einer Eosinophilenabnahme günstige Voraussetzungen für eine Prävention und Therapie von Restenose, In-Stent-Inflammation und Myokardinfarkt nach perkutaner Koronarintervention, Stentimplantation und koronararteriellem Bypass schaffen.

**Tabelle 1: Einfluss einer Behandlung mit dem Ginkgo biloba-Extrakt EGb 761® auf die Gesamtzahl weißer Blutkörperchen (WBC), sowie die Zahl der Monozyten (MON), Eosinophilen (EOS) und Lymphozyten (LYM) im peripheren Blut von Patienten mit metabolischem Syndrom**

| Patient | vorher | WBC [1/nL] nachher | Änderung [%] | vorher | MON [1/nL] nachher | Änderung [%] |
|---|---|---|---|---|---|---|
| 1 | 9.5 | 8.9 | -6.3 | 0.190 | 0.089 | -53.2 |
| 2 | 7.5 | 7.5 | 0.0 | 0.375 | 0.150 | -60.0 |
| 3 | 7.8 | 6.2 | -20.5 | 0.390 | 0.124 | -68.2 |
| 4 | 7.0 | 7.1 | +1.4 | 0.350 | 0.142 | -59.4 |
| 6 | 5.4 | 4.5 | -16.7 | 0.162 | 0.045 | -72.2 |
| 7 | 8.5 | 7.7 | -9.4 | 0.595 | 0.154 | -74.1 |
| 8 | 5.0 | 4.6 | -8.0 | 0.246 | 0.046 | -81.3 |
| 9 | 13.0 | 9.8 | -24.6 | 0.650 | 0.098 | -84.9 |
| 10 | 8.5 | 8.5 | 0.0 | 0.400 | 0.170 | -57.5 |
| 12 | 7.5 | 7.6 | +1.3 | 0.300 | 0.152 | -49.3 |
| 13 | 7.0 | 7.0 | 0.0 | 0.490 | 0.243 | -50.4 |
| Mean(11) | 7.9 | 7.2 | -7.5 | 0.377 | 0.128 | -64.6 |
| ± SEM | 0.6 | 0.5 | 2.8 | 0.047 | 0.017 | 3.7 |
| Median | | | -6.3 | | | -60.0 |
| *p* | | <0.052 | <0.024 | | <0.0001 | <0.001 |

| Patient | vorher | EOS [1/nL] nachher | Änderung [%] | vorher | LYM [1/nL] nachher | Änderung [%] |
|---|---|---|---|---|---|---|
| 1 | 0.095 | 0.089 | -6.3 | 4.1 | 3.5 | -14.6 |
| 2 | 0.075 | 0.075 | 0.0 | 2.6 | 2.7 | +3.9 |
| 3 | 0.234 | 0.124 | -47.0 | 2.3 | 2.0 | -13.0 |
| 4 | 0.210 | 0.071 | -66.2 | 2.1 | 2.3 | +9.5 |
| 6 | 0.054 | 0.045 | -16.7 | 1.6 | 1.5 | -6.3 |
| 7 | 0.340 | 0.077 | -77.4 | 2.5 | 2.0 | -20.0 |
| 8 | 0.123 | 0.046 | -62.6 | 1.3 | 1.8 | +38.5 |
| 9 | 0.260 | 0.098 | -62.3 | 2.5 | 2.9 | +16.0 |
| 10 | 0.160 | 0.170 | +6.3 | 2.7 | 2.3 | -14.8 |
| 12 | 0.225 | 0.076 | -66.2 | 2.9 | 3.0 | +3.5 |
| 13 | 0.280 | 0.081 | -71.1 | 2.6 | 2.7 | +3.9 |
| Mean(11) ± SEM | 0.187 | 0.087 | -42.7 | 2.5 | 2.4 | +0.6 |
| | 0.028 | 0.011 | 9.6 | 0.2 | 0.2 | 5.1 |
| Median *p* | | | -62.3 | | | +3.5 |
| | | <0.005 | <0.008 | | n.s. | n.s. |

Erklärung zur Tabelle 1:
- Mean:: arithmetischer Mittelwert
- SEM:: Standardfehler
- p:: Irrtumswahrscheinlichkeit
- vorher:: vor Behandlung (nach 4-wöchiger run-in-Phase)
- nachher:: nach 2-monatiger Behandlung (Medikation)
- Patienten 5 und 11:: wurden nach der Rekrutierung noch vor Studienbeginn ausgeschlossen, da sie unzuverlässig erschienen bzw. der Lp(a)-Wert zu niedrig war.

Gegenstand der Erfindung sind folglich Extrakte aus Blättern von Ginkgo biloba zur oralen Verwendung zur Prävention und Therapie von Komplikationen nach operativer Arteriosklerose-Behandlung. Die Extrakte können in Form eines Mittels verabreicht werden. Das Mittel kann dabei ein Arzneimittel oder ein Lebensmittel, wie z. B. ein Nahrungsergänzungsmittel (dietary supplement), ein funktionelles Lebensmittel (functional food, medical food), ein diätetisches Lebensmittel oder ein neuartiges Lebensmittel (novel food) sein.

Besondere Ausführungsformen der Erfindung sind nachstehend angegeben:
(1) Extrakt aus Blättern von Ginkgo biloba zur oralen Verwendung zur Prävention und Therapie von Komplikationen nach operativer Arteriosklerose-Behandlung, wobei es sich um die Beschleunigung der verzögerten Reendothelisierung nach der Implantation eines beschichteten Stents handelt, oder wobei es sich bei den Komplikationen nach operativer Arteriosklerose-Behandlung um Stentinduzierte Thrombosen oder um die Restenose von verengten Arterien nach der Implantation von unbeschichteten und beschichteten Stents handelt.
(2) Extrakt zur Verwendung nach (1), wobei der Extrakt Flavonoide und Terpenlactone enthält.
(3) Extrakt zur Verwendung nach (1) oder (2), wobei es sich bei dem Extrakt um einen Trockenextrakt handelt.
(4) Extrakt zur Verwendung nach einem der Absätze (1) bis (3), wobei der Extrakt mindestens 22.0 und höchstens 27.0 Gew.-% Flavonoide sowie mindestens 5.0 und höchstens 7.0 Gew.-% Terpenlactone enthält.
(5) Extrakt zur Verwendung nach einem der Absätze (1) bis (4), wobei der Extrakt 22.0 bis 27.0 Gew.-% Flavonoide und 2.6 bis 3.2 Gew.-% Bilobalid und 2.8 bis 3.4 Gew.-% Ginkgolide A, B und C (in der Summe) enthält.
(6) Extrakt zur Verwendung nach einem der Absätze (1) bis (5), wobei der Extrakt höchstens 5 ppm Ginkgolsäuren enthält.
(7) Extrakt zur Verwendung nach einem der Absätze (1) bis (6), wobei der Extrakt weniger als 10 ppm 4'-O-Methylpyridoxin enthält.
(8) Extrakt zur Verwendung nach einem der Absätze (1) bis (7), wobei die tägliche Dosierung 1 x 240 mg oder 2 x 120 mg beträgt.

Bevorzugt sind Ginkgo-Extrakte entsprechend den Vorgaben des DAB 2003 und des europäischen Arzneibuchs 6.1. Ginkgo-Extrakte gemäß DAB 2003 sind unter Verwendung von Aceton 60 % (m/m) und eines mehrstufigen Extraktionsverfahrens hergestellt, wobei das Verhältnis von Droge zu Extrakt (DEV) 35 bis 67 zu 1 beträgt und sind durch Gehalte an Flavonoiden von mindestens 22.0 % und höchstens 27.0 %, an Terpenlaktonen von mindestens 5.0 % und höchstens 7.0 % (davon 2.8 bis 3.4 % Ginkgolide A, B und C und 2.6 bis 3.2 % Bilobalid) und an Ginkgolsäuren von höchstens 5 ppm gekennzeichnet. Ginkgo-Extrakte gemäß Europäischem Arzneibuch 6.1 sind unter Verwendung von organischen Lösungsmitteln und deren Gemischen mit Wasser hergestellt, wobei das Verhältnis von Droge zu Extrakt (DEV) nicht näher angegeben ist und sind durch Gehalte an Flavonoiden von 22.0 % bis 27.0 %, an Bilobalid von 2.6 bis 3.2 %, an Ginkgoliden A, B und C von 2.8 bis 3.4 % und an Ginkgolsäuren von höchstens 5 ppm gekennzeichnet. Besonders bevorzugt ist der Ginkgo-Extrakt mit der Bezeichnung EGb 761®, der entsprechend dem DAB 2003 unter Verwendung von Aceton 60 % (m/m) und eines mehrstufigen Extraktionsverfahrens hergestellt ist, dessen Verhältnis von Droge zu Extrakt (DEV) 35 bis 67 zu 1 beträgt und der durch Gehalte an Flavonoiden von mindestens 22.0 % und höchstens 27.0 %, an Terpenlaktonen von mindestens 5.0 % und höchstens 7.0 % (davon 2.8 bis 3.4 % Ginkgolide A, B und C und 2.6 bis 3.2 % Bilobalid) und an Ginkgolsäuren von höchstens 5 ppm gekennzeichnet ist. Mit den genannten Eigenschaften entspricht EGb 761® auch dem Europäischen Arzneibuch 6.1. Auch wenn dies im DAB 2003 und im Europäischen Arzneibuch 6.1 nicht explizit angegeben ist, handelt es sich bei allen vorstehenden Prozentangaben um Gew.-%. Besonders bevorzugt sind auch Ginkgo-Extrakte entsprechend WO2006/117169, die darüber hinaus einen verminderten Gehalt an Biflavonen und/oder 4'-O-Methylpyridoxin (< 10 ppm) besitzen. Die Erfindung ist jedoch nicht auf die genannten Extrakte beschränkt.

Die Herstellung des besonders bevorzugten Ginkgo-Extraktes EGb 761® kann z. B. nach dem in EP431535B1 allgemein beschriebenen bzw. nach dem gemäß WO2006/117170 weiter optimierten Herstellungsverfahren erfolgen. Hierzu werden
(a) getrocknete und zerkleinerte Blätter von Ginkgo biloba bei einer Temperatur von etwa 40 bis 60 °C mit wässrigem Aceton (ca. 60 Gew.-% Aceton) extrahiert,
(b) das Aceton bis zu einem maximalen Gehalt von 5 Gew.-% abgetrennt,
(c) die verbliebene eingeengte wässrige Lösung mit Wasser bis zu einem Feststoffgehalt von ca. 10 Gew.-% verdünnt, bis auf eine Temperatur von höchstens 12 °C gekühlt und der resultierende Niederschlag entfernt,
(d) Ammoniumsulfat (ca. 30 Gew.-%) zur verbliebenen wässrigen Lösung zugefügt und die entstandene Lösung mit Methylethylketon oder einem Gemisch aus Methylethylketon und Aceton (6/4) extrahiert,
(e) die erhaltene organische Phase eingeengt und das so erhaltene Konzentrat mit Wasser und Ethanol so verdünnt, daß eine Lösung erhalten wird, die ca. 50 Gew.-% Wasser und 50 Gew.-% Ethanol bei einem Feststoffgehalt von ca. 10 Gew.-% enthält,
(f) eine wässrige Lösung von Bleihydroxidacetat zu der auf diese Weise erhaltenen Lösung zugefügt und der entstandene Niederschlag entfernt,
(g) die verbliebene wässrig-ethanolische Lösung mit Heptan extrahiert, um die Alkylphenolverbindungen weiter zu entfernen,
(h) die verbliebene wässrig-ethanolische Lösung unter vermindertem Druck eingeengt und ca. 20 Gew.-% Ammoniumsulfat zugefügt,
(i) die erhaltene Lösung mit einem Gemisch aus Methylethylketon und Ethanol (6/4) extrahiert,
(k) die resultierende organische Phase mit max. 20 Gew.-% Ammoniumsulfat getrocknet und eingeengt, so mit Ethanol versetzt, dass ein Ethanol-Gehalt von mindestens 80 Gew.-% resultiert, und 2 bis 10 h bei ≤ 12 °C gehalten und filtriert,
(l) das resultierende Filtrat unter vermindertem Druck eingeengt und getrocknet, wobei ein Trockenextrakt mit einem Wassergehalt von weniger als 5 % erhalten wird.

Zur Herstellung der besonders bevorzugten Ginkgo-Extrakte entsprechend WO2006/117169 mit einem verminderten Gehalt an Biflavonen und/oder 4'-O-Methylpyridoxin wird z. B. die Lösung aus obigem Schritt (k) gegebenenfalls mit wässrigem Ethanol verdünnt, über ein Adsorberharz und/oder einen Ionenaustauscher filtriert, wobei die zu entfernenden Stoffe auf dem Harz zurückgehalten werden, und unter vermindertem Druck zu einem Trockenextrakt mit einem Wassergehalt von weniger als 5 % getrocknet. Die Abreicherung von Biflavonen und/oder 4'-O-Methylpyridoxin führt dabei nicht zu einer signifikanten Beeinflussung der Gehalte an wirksamkeitsbestimmenden Komponenten.

Die erfindungsgemäß verwendbaren Ginkgo-Extrakte können in Form von Pulvern, Granulaten, Brausezubereitungen, Tabletten, Dragees, Kapseln oder Flüssigkeiten oral verabreicht werden. Zur Herstellung von Tabletten wird der Extrakt mit geeigneten pharmazeutisch verträglichen Hilfsstoffen wie z. B. Laktose, Cellulose, Siliciumdioxid, Croscarmellose und Magnesiumstearat gemischt und zu Tabletten gepresst, die gegebenenfalls mit einem geeigneten Überzug, z. B. aus Hydroxypropylmethylcellulose, Polyethylenglykol, Farbstoffen (z. B. Titandioxid), und Talkum versehen werden. Der erfindungsgemäße Extrakt kann auch, gegebenenfalls unter Zusatz von Hilfsstoffen wie z. B. Füllmittel, Fließregulierungsmittel etc., in Kapseln abgefüllt werden.

Bevorzugte Darreichungsform im Fall der Tabletten sind Filmtabletten. Ferner handelt es sich bei den bevorzugten Darreichungsformen um Formen, die den Extrakt im Körper schnell freisetzen und die im Europäischen Arzneibuch 6.0 als "Conventionalrelease dosage forms" bezeichnet werden.

## Patentansprüche

1. Extrakt aus Blättern von Ginkgo biloba zur oralen Verwendung zur Prävention und Therapie von Komplikationen nach operativer Arteriosklerose-Behandlung, wobei es sich um die Beschleunigung der verzögerten Reendothelisierung nach der Implantation eines beschichteten Stents handelt, oder wobei es sich bei den Komplikationen nach operativer Arteriosklerose-Behandlung um Stent-induzierte Thrombosen oder um die Restenose von verengten Arterien nach der Implantation von unbeschichteten und beschichteten Stents handelt.

2. Extrakt zur Verwendung nach Anspruch 1, wobei der Extrakt Flavonoide und Terpenlactone enthält.

3. Extrakt zur Verwendung nach Anspruch 1 oder 2, wobei es sich bei dem Extrakt um einen Trockenextrakt handelt.

4. Extrakt zur Verwendung nach einem der Ansprüche 1 bis 3, wobei der Extrakt mindestens 22.0 und höchstens 27.0 Gew.-% Flavonoide sowie mindestens 5.0 und höchstens 7.0 Gew.-% Terpenlactone enthält.

5. Extrakt zur Verwendung nach einem der Ansprüche 1 bis 4, wobei der Extrakt 22.0 bis 27.0 Gew.-% Flavonoide und 2.6 bis 3.2 Gew.-% Bilobalid und 2.8 bis 3.4 Gew.-% Ginkgolide A, B und C (in der Summe) enthält.

6. Extrakt zur Verwendung nach einem der Ansprüche 1 bis 5, wobei der Extrakt höchstens 5 ppm Ginkgolsäuren enthält.

7. Extrakt zur Verwendung nach einem der Ansprüche 1 bis 6, wobei der Extrakt weniger als 10 ppm 4'-O-Methylpyridoxin enthält.

8. Extrakt zur Verwendung nach einem der Ansprüche 1 bis 7, wobei die tägliche Dosierung 1 x 240 mg oder 2 x 120 mg beträgt.

## Claims

1. Extract made of leaves of Ginkgo biloba for oral use for the prevention or therapy of complications following surgical arteriosclerosis treatment, wherein an acceleration of delayed re-endothelialization after implantation of a coated stent occurs, or wherein the complications following surgical arteriosclerosis treatment is stent-induced thrombosis or restenosis of constricted arteries after implantation of uncoated and coated stents.

2. Extracts for use according to claim 1, wherein the extract contains flavonoids and terpene lactones.

3. Extract for use according to claim 1 or 2, wherein the extract is a dry extract.

4. Extract for use according to one of claims 1 to 3, wherein the extract contains at least 22.0 and at most 27.0 % by weight of flavonoids and at least 5.0 and at most 7.0 % by weight of terpene lactones.

5. Extract for use according to one of claims 1 to 4, wherein the extract contains from 22.0 to 27.0 % by weight of flavonoids, and 2.6 to 3.2 % by weight of bilobalide, and 2.8 to 3.4 % by weight of ginkgolides A, B, and C (as sum).

6. Extract for use according to one of claims 1 to 5, wherein the extract contains at most 5 ppm of ginkgolic acids.

7. Extract for use according to one of claims 1 to 6, wherein the extract contains less than 10 ppm 4'-O-methyl pyridoxine.

8. Extract for use according to one of claims 1 to 7, wherein the daily dosage is 1 x 240 mg or 2 x 120 mg.

## Revendications

1. Extrait de feuilles de *Ginkgo biloba* pour l'utilisation pour la prévention et la thérapie des complications consécutives à un traitement chirurgical de l'artériosclérose, dans lequel une accélération de la réendothélisation retardée apparait après l'implantation d'un stent revêtu, ou dans lequel les complications consécutives à un traitement chirurgical de l'artériosclérose sont des thromboses induites par un stent, ou la resténose des artères rétrécies après l'implantation de stents non-revêtus.

2. Extrait pour l'utilisation selon la revendication 1, dans lequel l'extrait contient des flavonoïdes et des lactones terpéniques.

3. Extrait pour l'utilisation selon la revendication 1 ou 2, l'extrait étant un extrait sec.

4. Extrait pour l'utilisation selon l'une des revendications 1 à 3, dans lequel l'extrait contient au moins 22,0 et au plus 27,0% en poids de flavonoïdes et au moins 5,0 et au plus 7,0% en poids de lactones terpéniques.

5. Extrait pour l'utilisation selon l'une des revendications 1 à 4, dans lequel l'extrait contient 22,0% à 27,0% en poids de flavonoïdes et 2,6% à 3,2% en poids de bilobalide et 2,8% à 3,4% en poids de ginkgolides A, B et C (au total).

6. Extrait pour l'utilisation selon l'une des revendications 1 à 5, dans lequel l'extrait contient au plus 5 ppm d'acides ginkgoliques.

7. Extrait pour l'utilisation selon l'une des revendications 1 à 6, dans lequel l'extrait contient moins de 10 ppm de 4'-O-méthyl-pyridoxine.

8. Extrait pour l'utilisation selon l'une des revendications 1 à 7, dans lequel le dosage quotidien est 1 x 240 mg ou 2 x 120 mg.
